# EUROPÄISCHE PATENTSCHRIFT

(11) **EP 0 729 762 B1**
(45) Veröffentlichungstag und Bekanntmachung des Hinweises auf die Patenterteilung: **04.12.2002**
(21) Anmeldenummer: 95102972.7
(22) Anmeldetag: 02.03.1995
(51) Int. Cl.: A61M 11/00, F04D 29/66, F04B 39/00

(54) **Inhalationsgerät mit integriertem Kompressorschalldämpfer**
Inhalation apparatus with integrated compressor sound absorber
Dispositif d'inhalation avec silencieux intégré au compresseur

(43) Veröffentlichungstag der Anmeldung: 04.09.1996
(73) Patentinhaber: PARI GmbH Spezialisten für effektive Inhalation, 82319 Starnberg (DE)
(72) Erfinder: Remke, Matthias, D 82319 Starnberg (DE); Brugger, Stephan, D 82301 Starnberg (DE)
(74) Vertreter: Zangs, Rainer E., Dipl.-Ing.

(56) Entgegenhaltungen:
- DE-A- 4 423 455
- FR-A- 2 604 093
- GB-A- 2 043 787
- US-A- 3 877 842
- US-A- 4 264 282

## Beschreibung

Die Erfindung betrifft ein Inhalationsgerät mit Vernebler - und mit Kompressoreinheit.

Aus FR 2.604.093 A ist ein Inhalationsgerät mit einer Verneblereinheit und einer Kompressoreinheit bekannt. Die Verneblereinheit dient zum Vernebeln einer Flüssigkeit durch zugeführte Komprimrerte Luft, die von der Kompressoreinheit gelietert wird. Diese besteht aus einem Gehäuse und einem darin angeordneten. Kompressor, der als geräuscharm berchrieben wird.

In GB 2 043 787 A ist eine Rotationspumpe beschrieben, mit der sich ein Luftbett oder Kissen fortlaufend mit Luft unter Druck versorgen lässt. Hierbei steht eine besonders geringe Lärmbelästigung im Vordergrund.

In US-A-3,877,842 wird vorgeschlagen, Luftpumpen mit Kanälen zum Führen von Kühlungsluft zu versehen. Die Kühlluft verläuft durch das Innere der Pumpe zum Unterstützen einer wirksamen Kühlung der Pumpe.

Weiterhin ist in US-A-4,264,282 ein Luftkompressorgerät mit einer lärmreduzierenden Vorrichtung zum Umsetzen von Umgebungsluft in komprimierte Luft beschrieben.

Im Gegensatz zu den vorangenannten Druckschriften ist es bei der Durchführung einer medizinischen Inhalationstherapie notwendig, einen flüssigen oder festen Wirkstoff so mit einem Gas zu vermischen, dass ein einatembares Aerosol entsteht. Ein sehr wirksames Verfahren ist die Vermischung von komprimierter Luft und einem flüssigen Wirkstoffträger mittels einer Dispergierdüse oder ähnlichen Vermischungseinrichtungen. Die Verwendung von auf einen definierten Druck komprimierter Luft erlaubt eine hohe Dosisgenauigkeit bei geeigneter Steuerung.

Bekannte Inhalationsgeräte besitzen eine Kompressoreinheit, die im wesentlichen aus dem eigentlichen Kompressor, der in einem Gehäuse untergebracht ist, einem an einem Luftansauganschluss angeordneten Filter, über den angesaugte

Luft dem Kompressor zugeführt wird, einem Luftauslassanschluss für komprimierte Luft und elektrischen Teilen zur Zuführung und Steuerung der für den Kompressor erforderlichen elektrischen Energie besteht. An den Luftauslassanschluss wird eine Verneblereinheit des Inhalationsgerätes angeschlossen, der die für die Zerstäubung des Medikaments erforderliche komprimierte Luft zugeführt wird.

In einigen Fällen ist das Gehäuse so ausgebildet, dass die Verneblereinheit daran angebracht werden kann, wodurch ein nicht nur für die Inhalationstherapie geeignetes, sondern auch ein kompaktes Inhalationsgerät entsteht.

Ein Problem bei den üblicherweise eingesetzten Kompressoren, insbesondere Membrankompresoren, besteht in der Geräuschentwicklung während des Betriebs, was einerseits zu einem unnötigen Leistungsverbrauch führt und andererseits für den Benutzer unangenehm ist, der das Inhalationsgerät in der Regel zur Behandlung einer akuten Erkrankung verwendet und der neben der durch die Krankheit hervorgerufenen psychischen Belastung nicht zusätzlich noch durch störende Geräusche des Inhalationgerätes belastet werden sollte. Ursache für die Geräusche sind vor allem Ansauggeräusche bei der Luftzuführung, insbesondere aufgrund der pulsierenden Förderung bei Membrankompressoren oder Kolbenkompressoren.

Zur Abschwächung der Ansauggeräusche ist es bekannt, am Ansaugfilter einen Filterdämpfer vorzusehen oder den Ansaugfilter selbst als Dämpfer auszugestalten. Es hat sich jedoch als problematisch erwiesen, daß für eine effiziente Dämpfung relativ große Schalldämpfer eingesetzt werden müssen, da der Schalldämpfer aufgrund seiner Größe wesentlich die Geräteabmessungen bestimmt bzw. einen großen Einfluß auf die Form und Gestaltung der Kompressoreinheit des Inhalationsgerätes hat. Insbesondere ist es erforderlich, den Ansaugwiderstand bei gleichzeitig ausreichender Dämpfung so gering wie möglich zu halten. Solche großen Dämpfer schaffen überdies Probleme bei der Herstellung der Geräte, da komplexe Gehäuse notwendig sind und beim Zusammenbau eine Vielzahl von Montageschritten, insbesondere bei der Herstellung der Strömungsverbindungen benötigt werden. Schließlich muß das Inhalationsgerät auch für den erkrankten Menschen handhabbar sein, d.h. es muß kompakt und so leicht wie möglich gestaltet sein.

Es ist Aufgabe der vorliegenden Erfindung, ein Inhalationsgerät zu schaffen, bei dem einerseits Kompressorgeräusche insbesondere Ansauggeräusche wirkungsvoll bei gleichzeitig geringem Ansaugwiderstand gedämpft sind, das aber andererseits kompakt gestaltet ist.

Gelöst wird die Aufgabe durch ein Inhalationsgerät mit den Merkmalen des Patentanspruchs 1. Vorteilhafte Ausgestaltungen ergeben sich aus den Unteransprüchen.

Da gemäß der vorliegenden Erfindung die Größe und Form des Schalldämpfungshohlraums an die Kompressorform angepaßt werden, werden die Gehäuseabmessungen praktisch allein vom eigentlichen Kompressor bestimmt. Der Ansaugwiderstand wird nur gering erhöht. In einer vorteilhaften Ausbildung hat die erfindungsgemäße Vorrichtung den Vorteil der leichten Gerätegehäuse-Montierfähigkeit, da bei der Geräteherstellung der Dämpfungshohlraum zunächst integral mit dem Kompressor, d.h. in räumlich-körperlicher Verbindung mit diesem angefertigt und anschließend der zusammengebaute Kompressor mit integriertem Kompressorschalldämpfer in das Gehäuse der Kompressoreinheit eingesetzt werden kann.

Es ist also zusammfassend festzustellen, daß die vorliegende Erfindung ein Inhalationsgerät mit einem kompakten und leicht zu montierenden Kompressor schafft, bei dem gleichzeitig eine effiziente Schalldämpfung verwirklicht ist. Das erfindungsgemäße Inhalationsgerät entwickelt beim Betrieb kaum vom Patienten als störend empfundene Geräusche; dabei ist aber die Druckluftleistung des Kompressors nicht wesentlich reduziert.

Weitere Merkmale und Vorteile der Erfindung können der nachfolgenden Beschreibung und den begleitenden Zeichnungen entnommen werden; es zeigt:
- Fig. 1: eine Perspektivansicht eines ersten Ausführungsbeispiels der Erfindung;
- Fig. 2: eine Darstellung eines zweiten Ausführungbeispiels der Erfindung; und
- Fig. 3: eine Innenansicht eines dritten Ausführungsbeispiels.

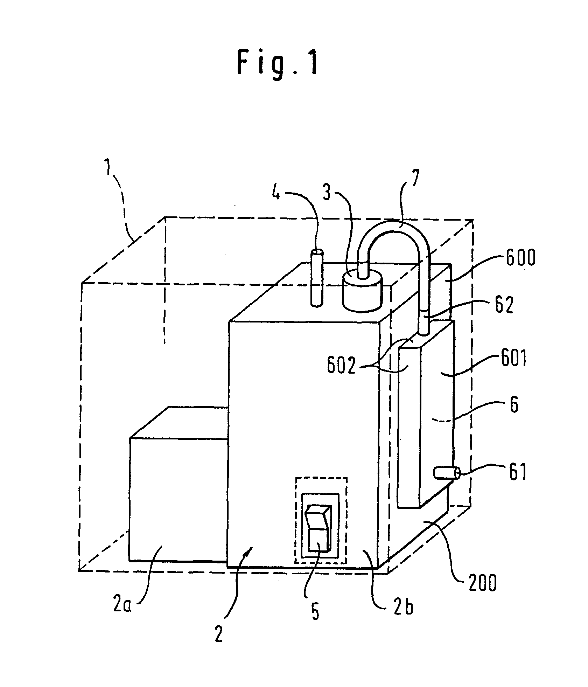

Fig. 1 zeigt ein erstes Ausführungsbeispiel der Erfindung, dessen grundsätzlicher Aufbau den Gedanken der Erfindung zum Ausdruck bringt. Das Problem der effizienten und kompakten Schalldämpfung von Kompressorgeräuschen wird gelöst durch Vorsehen eines mit dem Kompressor integral verbundenen Schalldämpfers, der aus einem Dämpfungshohlraum besteht, durch welchen die zum Filter oder Filterdämpfer am Luftansauganschluß geführte Luft geführt wird.

In Figur 1 sieht man eine Kompressoreinheit eines Inhalationsgerätes, im wesentlichen bestehend aus einem (gestrichelt dargestellten) Gehäuse 1 und einem darin aufgenommenen Kompressor 2. Der Kompressor 2, umfaßt einen Motor 2a, ein Pleuelgehäuse 2b und eine Kompressionseinrichtung, z.B. eine Membran oder einen Kolben. Der Motor treibt über ein Pleuel die Kompressionseinrichtung an. Weiterhin erkennt man einen Luftansauganschluß 3, an dem ein als Dämpfer wirkender Filter kompakter Bauart mit nur beschränktem Schalldämpfungsvermögen vorgesehen ist. An einem Luftauslaßanschluß 4 wird komprimierte Luft zur Zuführung zu einem Vernebler (nicht abgebildet) abgegeben. Weiterhin abgebildet ist ein Schalter 5, mit dem der Kompressor ein- und ausgeschaltet werden kann. Weitere Bestandteile der Energiezuführung zum Kompressor, beispielsweise der Anschluß an das Stromnetz, Sicherungen oder die Verkabelung sind zur Vereinfachung der Darstellung in Fig. 1 nicht abgebildet.

Erfindungsgemäß ist am Kompressor 2 ein Schalldämpfer bestehend aus einem Dämpfungshohlraum 6 vorgesehen, durch den die Luft zum Ansauganschluß 3 geführt wird. Die Luft tritt in eine Lufteinlaßöffnung 61 des ansonsten geschlossenen Dämpfungshohlraums 6 ein und wird über eine Schlauchverbindung 7 von einer Luftauslaßöffnung 62 des Dämpfungshohlraums zum Luftansauganschluß 3 geführt. Die Verbindung zwischen dem Dämpfungshohlraum und dem Kompressoreingang kann jedoch auch anders beispielsweise in Form einer im Kompressor oder im Kompressorgehäuse integrierten Luftführung gestaltet sein. Das Gehäuse 1 der Kompressoreinheit ist aufgrund der erfindungsgemäßen Gestaltung von Kompressor 2 und Schalldämpfer 6 sehr kompakt ausführbar.

Im Einzelnen wird der Dämpfungshohlraum 6 gebildet durch eine Bodenwand 600, die integral verbunden ist mit der Kompressorgehäusewand 200 oder die die Kompressorgehäusewand 200 bildet, einer Deckelwand 601 sowie einer Seitenwand 602, so daß bis auf die Lufteinlaßöffnung 61 und Luftauslaßöffnung 62 ein abgeschlossener Hohlraum entsteht. Die beiden großflächigen Wände 600 und 601 sind vorzugsweise an den Querschnitt des Kompressors 2, vorzugsweise des Pleuelgehäuses 2b angepaßt. Im Grenzfall stimmen die Außenkonturen des flachen Dämpfungshohlraums 6 mit den Konturen des Kompressors 2 bzw. des Pleuelgehäuses 2b überein. Erfindungsgemäß wird somit die Querschnittsfläche des Kompressors 2 ausgenutzt und eine sehr flache Bauform des Dämpfungshohlraums 6 ermöglicht.

Durch den Dämpfungshohlraum 6 im Zusammenwirken mit dem kleinen, dämpfenden Filter am Luftansauganschluß erzielt man insgesamt eine hohe Schalldämpfung, ohne daß das Gehäuse 1 sehr viel größer sein muß, als durch den Kompressor 2 vorgegeben wird. Der Dämpfungshohlraum 6 kann an eine beliebige Kompressorform angepaßt sein und durch seine Vorschaltung ist es möglich, den Filterdämpfer unmittelbar am Kompressoreingang klein auszuführen.

Vorzugsweise ist der Dämpfungshohlraum 6 am Pleuelgehäuse 2b anzuordnen, da das Pleuelgehäuse gewöhnlich der längste Teil eines Kompressors ist, so daß dadurch ein möglichst langer Dämpfungsweg für die zugeführte Luft erzielbar ist. Im Grenzfall stimmen die Außenkonturen von Dämpfungshohlraum 6 und Pleuelgehäuse 2b überein.

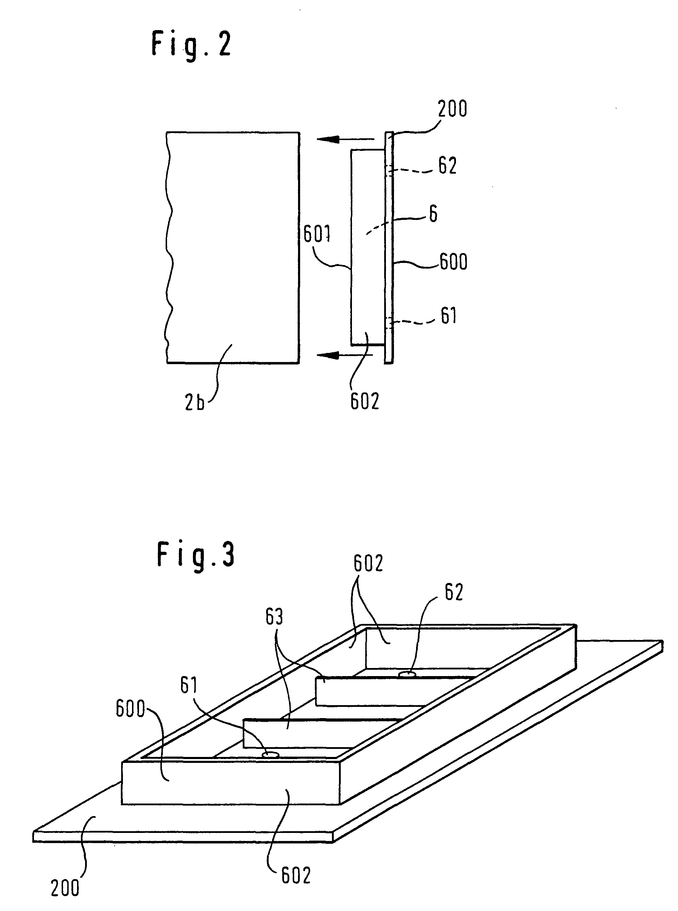

In Fig. 2 ist eine weitere Ausführung abgebildet, bei der der Dämpfungshohlraum 6 nicht außen am Pleuelgehäuse des Kompressors angeordnet ist, sondern sich ins Innere des Pleuelgehäuses 2b erstreckt. Der Dämpfungshohlraum 6 bildet eine Wand 200, die das Pleuelgehäuse 2b auf einer Seite abschließt. Hierdurch wird einerseits ein weiterer Raumgewinn erzielt und zusätzlich erreicht man durch die integrale Verbindung des doppelwandigen Dämpfungshohlraums mit dem Pleuelgehäuse eine Dämpfung von Pleuelgeräuschen. Bei diesem Ausführungsbeispiel wird eine gleichzeitige Dämpfung von Ansauggeräuschen und Pleuelgeräuschen erreicht.

Aufgrund der integrierten und damit in der Regel flachen Bauweise des Schalldämpfer wird bereits ein gewisses Schalldämpfungsvermögen erzielt. In einem bevorzugten Ausführungsbeispiel sind, wie in Fig. 3 gezeigt, in dem Dämpfungshohlraum 6 zwischen der Lufteinlaßöffnung 61 und der Luftauslaßöffnung 62 zusätzlich eines oder mehrere Stömungshindernisse 63 angeordnet, die dazu führen, daß die Luft nicht ungehindert zwischen Einlaß und Auslaß strömen kann. In der Figur ist die den Dämpfungshohlraum abschließende Deckelwand der Klarheit halber nicht abgebildet.

Die Ablenkung und die abwechselnde Verengung und Erweiterung der Strömungsführung verursacht eine Verwirbelung des Luftstromes und verhindert Resonanzerscheinungen, wodurch die Schalldämpfung gesteigert wird. Die Hindernisse 63 können, wie gezeigt, als Rippen ausgebildet sein, die senkrecht zur Verbindungslinie zwischen Einlaß 61 und Auslaß 62 angeordnet sind, wobei es vorteilhaft ist, die Rippen 63 gleich hoch auszuführen wie die Seitenwand 602, so daß die Rippen 63 ebenfalls die Bodenwand 600 und die Deckelwand 601 berühren. Die Luft vermag dann nur durch Lücken zu strömen, die vorgesehen sind zwischen den Rippen 63 und der Seitenwand 602 des Dämpfungshohlraums. Es ist aber auch denkbar, die Luftführung im Dämpfungshohlraum labyrinthartig vorzusehen.

Eine weitere bevorzugte Ausführung der Erfindung besteht in der Einrichtung des Dämpfungshohlraumes 6 auf einer Seite des Pleuelgehäuses 2b, so daß auf der dem Pleuelgehäuse abgewandten Seite weitere, insbesondere Funktionselemente der Kompressoreinheit angeordnet werden können. Dabei handelt es sich beispielsweise um elektrische Anschlüsse nach außen, wie Buchsen bzw. ein elektrischer Ein-/Ausschalter sowie innere elektrische Verbindungselemente, wie Kabel oder Zwischenschaltglieder zwischen äußerer Stromversorgung und dem Elektromotor des Kompressors. Dadurch erreicht man gleichzeitig eine effiziente Schalldämpfung sowohl bezüglich von Ansauggeräuschen als auch von Pleuelgeräuschen, und man hat gleichzeitig eine sehr effiziente Raumausnutzung, so daß das Gehäuse 1 (Fig. 1) nur unwesentlich größer sein muß als der Kompressor, insgesamt also ein sehr kompakter Aufbau erzielt wird.

Da die Form des Schalldämpfungshohlraumes vollkommen an die Kompressorform angepaßt werden kann, wird somit das Gehäuse praktisch allein von dem Kompressorabmessungen bestimmt. Durch die integrale Bauweise wird schlußendlich nur die Größe des Dämpfungshohlraums von der Größe des Kompressorgehäuses mitbestimmt.

Alle Ausführungsformen haben den Vorteil der leichten Gerätegehäuse-Montierfähigkeit, da bei der Geräteherstellung der Dämpfungshohlraum zunächst integral mit dem Kompressorgehäuse geschaffen wird und anschließend der zusammengebaute Kompressor in das Gerätegehäuse eingefügt wird.

## Patentansprüche

1. Inhalationsgerät mit
- einer Verneblereinheit, der die für die Zerstäubung eines Medikaments erforderliche komprimierte Luft zuführbar ist, und
- einer Kompressoreinheit, die
- ein Gehäuse (1),
- einen Kompressor (2), der in dem Gehäuse (1) aufgenommen ist und der einen Luftansauganschluss (3) und einen Luftauslassanschluss (4) aufweist, und
- einen Schalldämpfer (6), durch den Luft dem Luftansauganschluss (3) zugeführt wird, zur Dämpfung der Ansauggeräusche des Kompressors (2)
umfasst, wobei der Schalldämpfer (6) als Dämpfungshohlraum ausgebildet ist,
- der abgesehen von einer Lufteinlassöffnung (61) und einer Luftauslassöffnung (62) abgeschlossen ist und
- an einen Querschnitt des Kompressors (2) derart angepasst ist, dass die Außenkonturen des Dämpfungshohlraums im Grenzfall mit den Konturen des Kompressors übereinstimmen.

2. Inhalationsgerät nach Anspruch 1, **dadurch gekennzeichnet, daß** der Dämpfungshohlraum (6) integral mit dem Kompressor (2, 2a, 2b) verbunden ist.

3. Inhalationsgerät nach Anspruch 1 oder 2, **dadurch gekennzeichnet, daß** der Dämpfungshohlraum (6) sich in das Innere des Kompressors (2, 2a, 2b) erstreckt.

4. Inhalationsgerät nach Anspruch 1, 2 oder 3, **dadurch gekennzeichnet, daß** der Kompressor (2) ein Membrankompressor mit einem Pleuelgehäuse (2b) ist und der Dämpfungshohlraum (6) am bzw. im Pleuelgehäuse (2b) des Kompressors (2) angeordnet ist.

5. Inhalationsgerät nach einem der vorhergehenden Ansprüche, **dadurch gekennzeichnet, daß** die Verbindungslinie zwischen der Lufteinlaßöffnung (61) und der Luftauslaßöffnung (62) des Dämpfungshohlraums (6) parallel zu einer Längsachse des Kompressor (2, 2a, 2b) verläuft.

6. Inhalationsgerät nach einem der vorhergehenden Ansprüche, **dadurch gekennzeichnet, daß** zwischen der Lufteinlaßöffnung (61) und der Luftauslaßöffnung (62) Strömungshindernisse (63) im Dämpfungshohlraum (6) vorgesehen sind.

7. Inhalationsgerät nach Anspruch 6, **dadurch gekennzeichnet, daß** die Strömungshindernisse eine oder mehrere Rippen (63) sind, die senkrecht zur Verbindungslinie zwischen Lufteinlaßöffnung (61) und Luftauslaßöffnung (62) angeordnet sind.

8. Inhalationsgerät nach einem der vorhergehenden Ansprüche, **dadurch gekennzeichnet, daß** der Dämpfungshohlraum (6) die Wand des Kompressors bildet und auf der dem Kompressor abgewandten Seite weitere Funktionselemente des Kompressors angeordnet sind.

9. Inhalationsgerät nach Anspruch 8, **dadurch gekennzeichnet, daß** die weiteren Funktionselemente elektrische Funktionselemente sind.

10. Inhalationsgerät nach einem der vorhergehenden Ansprüche, **dadurch gekennzeichnet, daß** am Luftansauganschluß (3) des Kompressors (2, 2a, 2b) ein Filter oder Filterdämpfer vorgesehen ist, durch den die Luft vom Dämpfungshohlraum (6) dem Kompressor zugeführt wird.

## Claims

1. Inhalation device having
- a nebulizer unit to which the compressed air required for atomising a medicinal drug is suppliable and
- a compressor unit which comprises
- a housing (1),
- a compressor (2) which is accommodated in the housing (1) and which possesses an air intake connection (3) and an air outlet connection (4) and
- a sound absorber (6) through which air is fed to the air intake connection (3) for damping the intake noises from the compressor (2),
wherein the sound absorber (6) is constructed as a damping cavity,
- apart from an air inlet opening (61) and an air outlet opening (62) is sealed off and
- and is adapted to a cross-section of the compressor (2) in such a way that the external contours of the damping cavity in the limiting case coincide with the contours of the compressor.

2. Inhalation device according to Claim 1, **characterized in that** the damping cavity (6) is integrally connected to the compressor (2, 2a, 2b).

3. Inhalation device according to Claim 1 or2, **characterized in that** the damping cavity (6) extends into the interior of the compressor (2, 2a, 2b).

4. Inhalation device according to Claim 1, 2 or 3, **characterized in that** the compressor (2) is a diaphragm-type compressor having a connecting rod housing (2b) and the damping cavity (6) is arranged on or in the connecting rod housing (2b) of the compressor (2).

5. Inhalation device according to one of the preceding claims, **characterized in that** the connecting line between the air inlet opening (61) and the air outlet opening (62) of the damping cavity (6) runs parallel to a longitudinal axis of the compressor (2, 2a, 2b).

6. Inhalation device according to one of the preceding claims, **characterized in that** between the air inlet opening (61) and the air outlet opening (62) obstacles to flow (63) are provided in the damping cavity (6).

7. Inhalation device according to Claim 6, **characterized in that** the flow obstacles are one or more ribs (63) which are arranged perpendicularly to the connecting line between the air inlet opening (61) and air outlet opening (62).

8. Inhalation device according to one of the preceding claims, **characterized in that** the damping cavity (6) forms the wall of the compressor and on the side facing away from the compressor further functional elements of the compressor are arranged.

9. Inhalation device according to Claim 8, **characterized in that** the further functional elements are electric functional elements.

10. Inhalation device according to one of the preceding claims, **characterized in that** at the air intake connection (3) of the compressor (2, 2a, 2b) a filter or filter damper is provided through which the air is fed from the damping cavity (6) to the compressor.

## Revendications

1. Dispositif d'inhalation avec
- une unité de vaporisation à laquelle peut être amené de l'air comprimé nécessaire à la vaporisation d'un médicament, et
- une unité de compression qui comprend :
- un boîtier (1)
- un compresseur (2) qui est contenu dans le boîtier (1) et qui présente une prise d'aspiration d'air (3) et un orifice d'échappement d'air (4), et
- un silencieux (6) par lequel l'air est amené à la prise d'aspiration d'air (3), pour l'atténuation des bruits d'aspiration du compresseur (2),
le silencieux (6) étant formé comme un vide d'amortissement fermé, mis à part un orifice de prise d'air et un orifice d'échappement d'air, et adapté à une section du compresseur (2) de telle sorte que les contours extérieurs du vide d'amortissement correspondent en cas limite aux contours du compresseur.

2. Dispositif d'inhalation selon la revendication 1, **caractérisé en ce que** le vide d'amortissement (6) est raccordé intégralement au compresseur (2, 2a, 2b).

3. Dispositif d'inhalation selon la revendication 1 ou 2, **caractérisé en ce que** le vide d'amortissement (6) s'étend à l'intérieur du compresseur (2, 2a, 2b).

4. Dispositif d'inhalation selon la revendication 1, 2 ou 3, **caractérisé en ce que** le compresseur (2) est un compresseur à membrane avec un boîtier de bielle (2b) et **en ce que** le vide d'amortissement (6) est placé sur ou dans le boîtier de bielle (2b) du compresseur (2).

5. Dispositif d'inhalation selon l'une des revendications précédentes, **caractérisé en ce que** la ligne de raccord entre l'orifice d'admission d'air (61) et l'orifice d'échappement d'air (62) du vide d'amortissement (6) est parallèle à un axe longitudinal du compresseur (2, 2a, 2b).

6. Dispositif d'inhalation selon l'une des revendications précédentes, **caractérisé en ce que** des obstacles d'écoulement (63) sont prévus dans le vide d'amortissement (6) entre l'orifice d'admission d'air (61) et l'orifice d'échappement d'air (62).

7. Dispositif d'inhalation selon la revendication 6, **caractérisé en ce que** les obstacles d'écoulement sont une ou plusieurs nervures (63) placées perpendiculairement à la ligne de raccord entre l'orifice d'admission d'air (61) et l'orifice d'échappement d'air (62).

8. Dispositif d'inhalation selon l'une des revendications précédentes, **caractérisé en ce que** le vide d'amortissement (6) forme la paroi du compresseur et **en ce que** d'autres éléments de fonctionnement du compresseur sont placés sur le côté détourné du compresseur.

9. Dispositif d'inhalation selon la revendication 8, **caractérisé en ce que** les autres éléments de fonctionnement sont des éléments de fonctionnement électriques.

10. Dispositif d'inhalation selon l'une des revendications précédentes, **caractérisé en ce qu**'un filtre ou un amortisseur de filtre est prévu sur la prise d'aspiration d'air (3) du compresseur (2, 2a, 2b), par lequel l'air est amené par le vide d'amortissement (6) du compresseur.
